# EUROPEAN PATENT APPLICATION

(11) **EP 4 650 339 A2**
(43) Date of publication of application: **19.11.2025**
(21) Application number: 25172593.3
(22) Date of filing: 25.04.2025
(51) Int. Cl.: C07C 29/151

(54) **ADVANCED CONTROL AND RELATED METHODS FOR A LOW CARBON METHANOL FACILITY**

(30) Priority: 25.04.2024 US 202463638633 P; 14.05.2024 US 202463647504 P
(71) Applicant: Kellogg Brown & Root LLC, Houston, Texas 77002 (US)
(72) Inventor: PACHPANDE, Sunil Nivrutti, Houston, TX, 77002 (US); BANTWAL BALIGA, Satish, Houston, TX, 77002 (US); FENG, Zhentao, Houston, TX, 77002 (US); YAMALIDOU, Ekaterini, Houston, TX, 77002 (US)
(74) Representative: Weickmann & Weickmann PartmbB

(57) **Abstract**

Advanced control and related methods for a low carbon methanol facility having an methanol synthesis plant that includes systems to supply energy to the facility, wherein at least a portion of the supplied energy is from a low carbon energy source, receiving a forecasted energy profile for the low carbon energy source over a time period, predicting, using an advanced regulatory controller (ARC), the operating conditions of the facility based on the forecasted energy profile for the low carbon energy source, generating, by the ARC, one or more set points to control the facility; controlling the generating of the hydrogen feed using the ARC; and producing methanol by feeding the generated hydrogen feed to the methanol synthesis plant in accordance with one or more set points generated by the ARC.

## Description

### CROSS-REFERENCE TO RELATED APPLICATIONS

This application claims the benefit of U.S Provisional Application No. 63/638,633, filed on April 25, 2024, and of U.S. Provisional Application No. 63/647,504, filed on May 14, 2024, both of which are incorporated herein by reference in their entirety.

### TECHNICAL FIELD

The present disclosure relates to advanced control and related methods for a low carbon methanol facility.

### BACKGROUND

Conventionally, methanol is produced by reacting hydrogen with carbon dioxide in the presence of a catalyst. A hydrocarbon feedstock is usually used to generate the hydrogen feed for the process to produce methanol, and the generation of hydrogen from hydrocarbon feedstock is carbon intensive. Proposals to eliminate the hydrocarbon feedstock and decarbonize methanol production sometimes employ alternative hydrogen sources such as an electrolyzer/electrolysis process, which only requires water and electricity. If a renewable energy source supplies the electricity for the electrolyzer, then the hydrogen for methanol production can be generated without carbon emissions. However, because efficient methanol production requires steady-state conditions such as invariant flowrate for the hydrogen feed, using renewable energy sources can be problematic when operating an electrochemical system such as an electrolyzer.

Renewable energy sources, such as wind or solar power, are prone to changes in environmental conditions; e.g., lulls in winds, inclement weather, etc. A reduction in wind speed or sunlight intensity can lead to reductions in available electrical power. Reduced electrical power, in turn, causes the hydrogen production source (e.g., electrolyzer) to generate less hydrogen feed, which can limit methanol production.

The present disclosure addresses the need for efficient low carbon methanol production utilizing renewable energy sources. The present disclosure also addresses the need for systems and methods that allow a dynamic energy source, such as renewable energy, to provide energy to any process whose stability is affected by a dynamic energy supply.

### SUMMARY

In aspects, the present disclosure provides advanced control and related methods for a low carbon methanol facility that may address one or more problems of the prior art. In examples, the low carbon methanol facility may include a methanol synthesis plant composed of a carbon dioxide conditioning unit, a syngas compression unit, a methanol synthesis unit and a methanol distillation unit.

In examples, disclosed is a method for producing methanol using a low carbon energy source, the methanol being produced by a facility having a methanol synthesis plant, the method that may include supplying energy to the facility, wherein at least a portion of the supplied energy may be from a low carbon energy source; receiving a forecasted energy profile for the low carbon energy source over a time period; predicting, using an advanced regulatory controller (ARC), operating conditions of the facility based on the forecasted energy profile for the low carbon energy source; generating, by the ARC, one or more set points to control the facility; generating a hydrogen feed to the methanol synthesis plant using at least one of: (i) a primary hydrogen feed generated by a hydrogen plant energized by the low carbon energy source, and (ii) a supplemental hydrogen feed; controlling the generating of the hydrogen feed using the ARC; and producing methanol by feeding the generated hydrogen feed to the methanol synthesis plant in accordance with one or more set points generated by the ARC.

In examples, generating the hydrogen feed may include using the supplemental hydrogen feed provided from: (i) a hydrogen storage unit energized by the low carbon energy source, (ii) a secondary hydrogen source, or (iii) a combination of (i) and (ii).

In examples, the one or more set points for the facility may be generated using facility-specific information and non-facility-specific information, the non-facility-specific information that may include the forecasted energy profile of the low carbon energy source.

In examples, the non-facility-specific information further may include availability of energy from a secondary energy source, and further that may include transferring energy between the facility and the secondary energy source.

In examples, the transferring may include one of: (i) exporting energy from the low carbon energy source to the secondary energy source, and (ii) importing energy from the secondary energy source to the facility.

In examples, the forecasted energy profile for the low carbon energy source may be received continuously or periodically.

In examples, the one or more set points may be generated by the ARC each time an update to the energy profile for the low carbon energy source may be received.

In examples, the method may include determining if the predicted operating conditions of the facility may be outside operating limits of one or more sections or equipment of the facility.

In examples, generating the one or more set points for the facility further may include: determining that the predicted operating conditions of the facility may be outside the operating limits of one or more sections or equipment of the facility; determining, in response to the determination that the predicted operating conditions may be outside the operating limits, a maximum hydrogen rate that may be consumed by the methanol synthesis plant without violating any of the operating limits of any equipment and/or process in the facility; and generating the one or more set points for the facility based on the determined maximum hydrogen rate and on the operating limits of one or more sections or equipment of the facility.

In examples, the method may include converting the forecasted energy profile for the low carbon energy source into a net target hydrogen flow amount; wherein predicting operating conditions may be based at least in part on the net target hydrogen flow amount; and wherein the one or more set points may be generated based at least in part on the operation conditions predicted based on the net target hydrogen flow amount.

In examples, disclosed is a control system for producing methanol using a low carbon energy source, the methanol being produced by a facility having a methanol synthesis plant, the control system that may include: an advanced regulatory controller (ARC) configured to: receive a forecasted energy profile for the low carbon energy source over a time period; predict operating conditions of the facility based on the forecasted energy profile for the low carbon energy source; and generate one or more set points for the facility and to control the generation of a hydrogen feed to the methanol synthesis plant for production of the methanol.

In examples, the one or more set points may be generated based on the received forecasted energy profile for the low carbon energy source.

In examples, the ARC may be configured to receive a level of energy availability from a secondary energy source.

In examples, the ARC may be configured to cause a transfer of energy between the facility and the secondary energy source.

In examples, the ARC may be configured to: determine if the predicted operating conditions of the facility may be outside operating limits of one or more sections or equipment of the facility; determine, in response to the determination that the predicted operating conditions of the facility may be outside the operating limits, a maximum hydrogen rate that may be consumed by the methanol synthesis plant without violating any of the operating limits of any equipment and/or process in the facility; and use the maximum hydrogen rate and on the operating limits of one or more sections or equipment of the facility to generate one or more set points.

In examples, the control system may include one or more distributed control systems configured to receive the one or more set points generated by the ARC.

In examples, the ARC may include a subsystem configured to convert the forecasted energy profile for the low carbon energy source into a net target hydrogen flow amount; wherein the operating conditions may be predicted based on the net target hydrogen flow amount; and wherein the one or more set points may be generated based on the operation conditions predicted based on the net target hydrogen flow amount.

In examples, disclosed is a non-transitory computer readable medium having stored thereon computer-readable instructions that, when executed by a processor, cause the processor to receive facility-specific information and non-facility-specific information, wherein the non-facility-specific information may include a forecasted energy profile for a low carbon energy source over a time period; predict operating conditions of the facility based on the forecasted energy profile for the low carbon energy source; determine if the predicted operating conditions of the facility may be outside operating limits of one or more sections or equipment of the facility; generate, based on the determination, one or more set points for the facility; and control production of methanol based on one or more set points.

In examples, the non-transitory computer readable medium may be configured to continuously monitor one or more components of a facility that may include a methanol synthesis plant and the low carbon energy source.

In examples, the non-transitory computer readable medium may be configured to receive a level of energy availability from a secondary energy source.

In examples, the non-transitory computer readable medium may be configured to cause a transfer of energy from the low carbon energy source to a secondary source of energy.

In examples, the non-transitory computer readable medium may be configured to: convert the forecasted energy profile for the low carbon energy source into a net target hydrogen flow amount; wherein the operating conditions may be predicted based at least in part on the net target hydrogen flow amount; and wherein the one or more set points may be generated based at least in part on the operation conditions predicted based on the net target hydrogen amount.

In examples, disclosed is a method for controlling a facility for producing methanol using a low carbon energy source and a methanol synthesis plant, that may include: receiving facility-specific information and non-facility-specific information, wherein the non-facility-specific information may include a forecasted energy profile for a low carbon energy source over a time period; predicting operating conditions of the facility based on the forecasted energy profile for the low carbon energy source; determining if the predicted operating conditions of the facility may be outside operating limits of one or more sections or equipment of the facility; generating, based on the determination, one or more set points for the facility; and controlling production of methanol based on one or more set points.

In examples, the method my include converting the forecasted energy profile for the low carbon energy source into a net target hydrogen flow amount, wherein the operating conditions may be predicted based at least in part on the net target hydrogen flow amount, and wherein the one or more set points may be generated based at least in part on the operation conditions predicted based on the net target hydrogen flow amount.

It should be understood that certain features of the disclosure have been summarized rather broadly in order that the detailed description thereof that follows may be better understood, and in order that the contributions to the art may be appreciated. There are, of course, additional features of the disclosure that will be described hereinafter and which will in some cases form the subject of the claims appended thereto.

### BRIEF DESCRIPTION OF THE DRAWINGS

For detailed understanding of the present disclosure, references should be made to the following detailed description taken in conjunction with the accompanying drawings, in which like elements have been given like numerals and wherein:
FIG. 1 schematically illustrates a low carbon process for synthesizing methanol using a low carbon energy source in accordance with one embodiment of the present disclosure;
FIG. 2 schematically illustrates an advanced regulatory controller (ARC) in accordance with one embodiment of the present disclosure configured to control a methanol synthesis facility that uses a low carbon energy source; and
FIG. 3 schematically illustrates a valve system for a hydrogen source used in the FIG. 1 embodiment.
FIG. 4 illustrates a flow diagram of an example of the process of the ARC as described herein.
FIG. 5 shows the description of an example of the method carried out by subsystem 150.
FIG. 6 shows the pressure profile of hydrogen in hydrogen storage unit 112 for iterative values of the target hydrogen flow for a power availability profile for a sample 24-hour horizon.
FIG. 7 shows the safety margin applied to the high and low operating pressure limits of the hydrogen storage unit 112.

### DETAILED DESCRIPTION

In aspects, the present disclosure provides systems and related methods for advanced control and related methods for a low carbon methanol facility. There are shown in the drawings, and herein will be described in detail, specific embodiments of the present disclosure with the understanding that the present disclosure is to be considered an exemplification of the principles of the disclosure and is not intended to limit the disclosure to that illustrated and described herein.

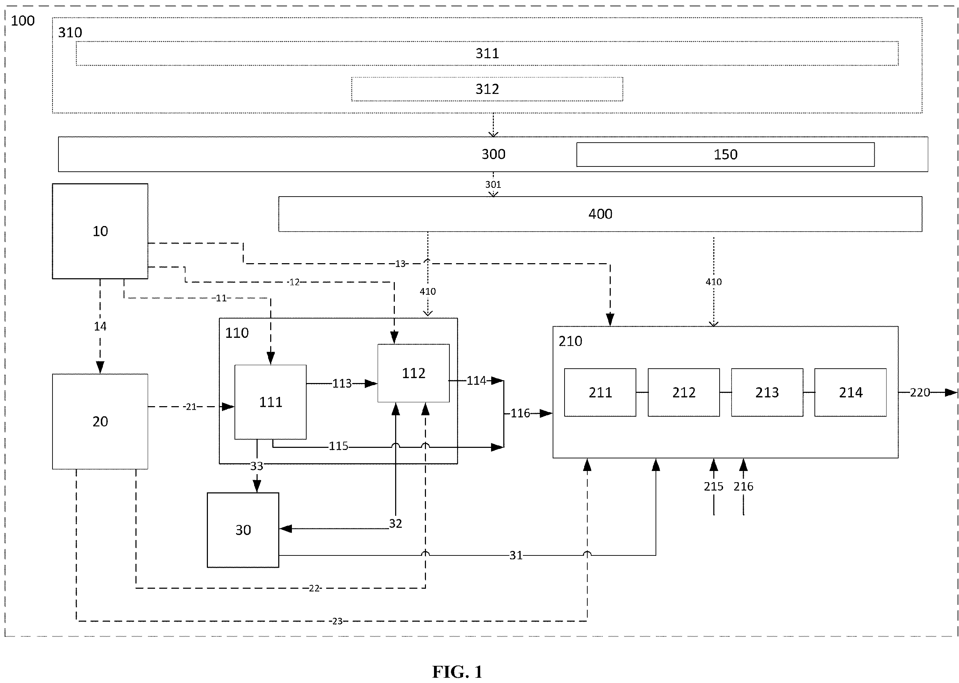

Referring to FIG. 1, there is schematically shown a non-limiting embodiment of a low carbon methanol production facility 100, hereafter 'facility 100,' in accordance with the present disclosure. Facility 100 includes a methanol synthesis plant 210 that produces a methanol product 220. The methanol synthesis plant is made of a carbon dioxide conditioning unit 211, a syngas compression unit 212, a methanol synthesis unit 213 and a methanol distillation unit 214.

To reduce or eliminate carbon emissions, a low carbon energy source 10 may be used to supply electrical energy to one or more components of the facility 100. Because the power output of the low carbon energy source 10 may fluctuate due to external influences, such as weather conditions, the supply of power from the low carbon energy source 10 may encounter prolonged interruptions or be subject to diminished capacity. With the benefit of the present teachings, such low carbon energy sources 10 may still be used to supply energy to the facility 100. Accordingly, it is emphasized that terms such as "supplying power" or "supplying energy" does not require an uninterrupted supply or power having any specified minimum requirements.

The methanol synthesis plant 210 may receive a biogenic carbon dioxide feed 215 or a non-biogenic carbon dioxide feed 216 and a direct hydrogen feed 115.

In examples, a secondary energy source 20 (e.g. power grid) may be available to provide energy to facility 100. The secondary energy source 20 is operationally independent of facility 100. That is, the secondary energy source 20 is not controlled by or dependent on facility 100.

In examples, facility 100 includes a hydrogen plant 110. In examples, the hydrogen plant 110 generates the direct hydrogen feed 115 using a low carbon process. Electricity for the process is supplied by the low carbon energy source 10. In one arrangement, the hydrogen plant 110 includes a primary hydrogen source 111 and a hydrogen storage unit 112. In one embodiment, the primary hydrogen source 111 may be an electrolyzer. In examples, the primary hydrogen source 111 may include an electrolyzer that generates a hydrogen feed that may include a direct hydrogen feed 115 to the methanol synthesis plant 210 and/or a storage hydrogen feed 113 to the hydrogen storage unit 112. The direct hydrogen feed 115 and the storage hydrogen feed 113 are shown as separate merely for clarity. A common effluent line (not shown) from the electrolyzer 111 used as primary hydrogen source may be used to selectively direct flow to either or both the methanol synthesis plant 210 and the hydrogen storage unit 112. It should be noted that the present teachings are not limited to a hydrogen plant 110 that uses only an electrolyzer 111 as primary hydrogen source to generate the hydrogen feed. The present teachings are equally applicable to any system or method of generating hydrogen via a low carbon process.

The hydrogen storage unit 112 provides a supplemental hydrogen feed for the methanol synthesis plant 210. In one arrangement, the hydrogen storage unit 112 stores hydrogen and supplies the stored hydrogen feed 114 to the methanol synthesis plant 210, if needed. Valves (not shown) can control the storage hydrogen feed 113 from the primary hydrogen source 111, such as an electrolyzer, to the hydrogen storage unit 112. Valves (not shown) can also control the stored hydrogen feed 114 from the hydrogen storage unit 112 to the methanol synthesis plant 210. In some embodiments, the hydrogen storage unit 112 may be supplied by a secondary hydrogen source 30 via a secondary hydrogen feed 32. Also, excess hydrogen from the primary hydrogen source 111 or hydrogen storage unit 112 may be exported to the secondary hydrogen source 30. The secondary hydrogen source 30 may also provide a supplemental hydrogen feed to the methanol synthesis plant 210. For example, hydrogen can be supplied to the methanol synthesis plant 210 directly from the secondary hydrogen source 30 via the direct secondary hydrogen feed 31. As used herein, a "secondary hydrogen source" is a hydrogen source that is operationally independent of facility 100. That is, the secondary hydrogen source has access to sources for receiving power and hydrogen that are independent of facility 100.

Facility 100 also includes an advanced regulatory controller (hereafter, 'ARC') 300 that determines one or more set points 301 for controlling one or more operations of facility 100. The ARC 300 may determine the set point(s) 301 for facility 100, based on forecasted energy profile for the low carbon energy source 10 over a time period and/or the energy availability from the secondary energy source 20. In examples, the time period may be a selected or preselected time period. In examples, the forecasted energy profile for the low carbon energy source 10 may be provided by the operator of the low carbon energy source 10, for example the operator of the solar farm, wind farm, tidal power plant, geothermal power plant, hydroelectric power plant, nuclear power plant, or hydrogen pipeline. In examples, the energy availability from the secondary energy source 20 may be provided by the operator of the secondary energy source 20. As used herein, a 'set point' is a value associated with a desired output, response, behavior, or operating state of a process component. A set point may be a value, range of values, an upper limit or a lower limit. By 'control,' it is meant modulating, stopping, starting, adjusting, increasing, decreasing, and/or maintaining one or more states, conditions, and/or parameters relating to a given operation. As further described below, the set point(s) 301 are transmitted to the distributed control systems (hereafter 'DCS') 400 of the facility 100.

The ARC 300 may include logic, computations, algorithms, schemas, microprocessors, memory modules, bi-directional signal transmission devices, display devices, input devices, and other components suitable for receiving, processing, storing, and transmitting information.

The ARC 300 determines the set points 301 using information 310, which may include facility-specific information 311 and/or non-facility-specific information 312. Facility-specific information 311, which is information relevant to the operation of facility 100, may include operating parameters and conditions of the equipment or components of the facility 100, as well as current set points of the primary hydrogen source 111 (e.g. an electrolyzer), biogenic carbon dioxide feed 215, non-biogenic carbon dioxide feed 216, hydrogen storage unit 112, methanol synthesis plant 210 and/or other component(s) associated with facility 100. Facility-specific information 311 may also include operating parameters such as pressure, temperature, flow rates, energy usage, etc. of one or more components of facility 100. The non-facility-specific information 312, which is information external to the operation of facility 100, may include information relating to the availability and demand of external resources, and other information that is independent of the operation of the facility 100. In examples, non-facility-specific information 312 may include the forecasted profile of energy for the low carbon energy source over a given period of time, availability of secondary energy source 20, availability of secondary hydrogen source 30, etc. It should be noted that the facility-specific information and the non-facility-specific information described above are only illustrative. The design and configuration of a facility 100, the geographical location in which the facility 100 is located, and the infrastructure in the vicinity of the facility 100 may require facility-specific information and/or non-facility-specific information that are not expressly listed above.

In examples, the ARC 300 is designed and configured to receive as an input the forecasted energy profile for the low carbon energy source 10 over a time period, which is the amount of energy available to facility 100 over the given period of time. The forecasted energy profile for the low carbon energy source 10 may be obtained from the operator of the low carbon energy source 10, for example the operator of the solar farm, wind farm, tidal power plant, geothermal power plant, hydroelectric power plant, nuclear power plant, or hydrogen pipeline. The forecasted energy profile for the low carbon energy source 10 over a time period is then used to determine one or more set points 301 of facility 100.

Illustrative, but not exhaustive, changes that may be made / recommended by the ARC 300 include increasing or decreasing the flow of hydrogen from the primary hydrogen source 111 (e.g. an electrolyzer) to the hydrogen storage unit 112, increasing or decreasing the flow of hydrogen from the primary hydrogen source 111 (e.g. an electrolyzer) to the methanol synthesis plant 210, and/or increasing or decreasing the flow of hydrogen from the hydrogen storage unit 112 to the methanol synthesis plant 210.

In examples, the ARC 300 establishes the target set points 301 for facility 100 aiming to keep the methanol synthesis plant operating in a stable manner, even when the forecasted energy profile for the low carbon energy source 10 shows high degree of variability over a period of time. When establishing the target set points 301, the ARC 300 may take into account one or more of facility-specific information 311 such as the minimum power requirements for the operation of all equipment of facility 100, the operating limits of all equipment of facility 100, including the allowable rate of change and the minimum turndown ratio of the methanol synthesis plant 210, and the availability of hydrogen in the hydrogen storage unit 112. When establishing the target set points 301, the ARC 300 may also take into account one or more of non-facility-specific information 312, such as the availability of power from the secondary energy source 20, the availability of hydrogen from the direct secondary hydrogen feed 115 and the requirements for the methanol product 220.

In examples, the ARC 300 receives the forecasted energy profile for the low carbon energy source 10 over a period of time, for example the next six hours and then the ARC 300 may calculate the cumulative availability of energy over the same time period.

In examples, the ARC 300 performs calculations using first principles model related to the operation of each equipment of facility 100 to determine the expected energy consumption based on the current operating conditions over the period of time equal to the length of the forecasted energy profile for the low carbon energy source 10. This calculation may be performed continuously and/or at predetermined time intervals, for example hourly, as well as each time the ARC 300 may receive an updated forecasted energy profile for the low carbon energy source 10. The ARC 300 may perform the calculations in stages, as illustrated below.

In examples, as shown in FIG. 1, ARC 300 includes a subsystem 150. In examples, subsystem 150 may be configured to generate the target hydrogen flow to the methanol synthesis plant 210, based on predicted operating conditions and given the availability of hydrogen. In examples, subsystem 150 may be implemented in software, hardware, or a combination of both. In examples, subsystem 150 may share one or more other components of ARC 300.

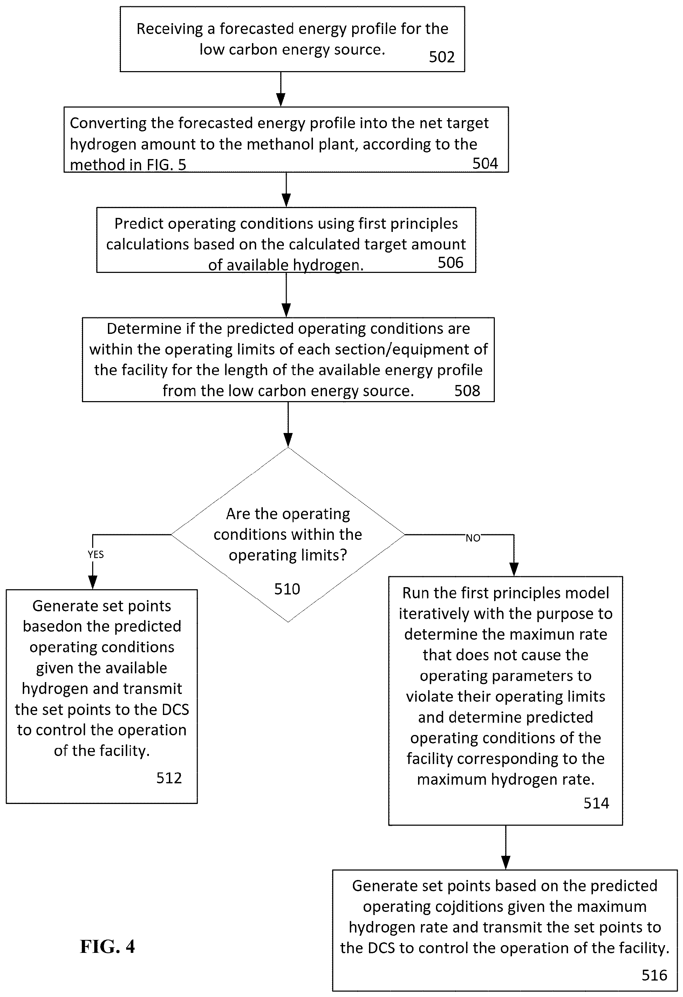

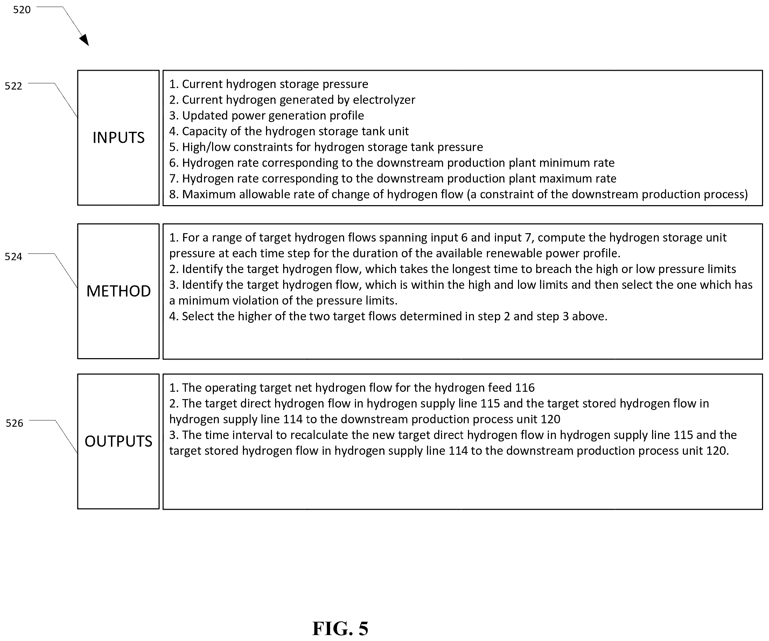

FIGS. 4 and 5 illustrate an example of the operation of ARC 300 including subsystem 150. In examples, the process of the operation of ARC 300 as for example, illustrated in FIGS. 4 and 5 may be repeated each time the ARC receives a new forecasted energy profile for the low carbon energy source.

As shown, for example in FIG. 4, at the start of the process, at 502, the ARC receives a forecasted energy profile for the low carbon energy source.

After receipt of the forecasted energy profile, at 504 (step 1) the ARC 300 may convert the forecasted energy profile for the low carbon energy source 10 into the cumulative amount of available hydrogen for the direct hydrogen feed 115 and the amount of available hydrogen of the stored hydrogen feed 114 to the methanol synthesis plant 210. The conversion takes into consideration the expected performance of the primary hydrogen source 111 (e.g. an electrolyzer), the power consumption by the methanol synthesis plant 210, the availability of biogenic carbon dioxide feed 215 and non-biogenic carbon dioxide feed 216 and other parts of the plant, for example the hydrogen storage unit 112, etc.

In examples, after receipt of the forecasted energy profile, at 504 the ARC 300 may trigger subsystem 150 to convert the forecasted energy profile for the low carbon energy source 10 into the target value for the net hydrogen flow 116 to the methanol synthesis plant 210 using the method described in FIG. 5.

In examples, subsystem 150 carries out iterative calculations to determine the current and future values of the pressure inside the hydrogen storage unit 112 across the time horizon for which the renewable power availability profile is given. In examples, using the current and future values of the pressure of the hydrogen storage unit 112 across the time horizon for which the renewable power variability profile, subsystem 150 may determine the target net hydrogen flow to the methanol synthesis plant 210 for the given renewable power availability profile and subsequently the target direct hydrogen flow in hydrogen supply line 115 and the target stored hydrogen flow in hydrogen supply line 114.

In examples, subsystem 150 is configured to perform a rolling and dynamic calculation of the amount of hydrogen generated by the primary hydrogen source 111 at certain frequency across the duration of the renewable power availability profile. In examples, any frequency may be used that is not longer than the time step by which the power availability profile is renewed. This frequency should be long enough to maintain the operation of the downstream production process as stable as possible. For purposes of this description, this frequency may be assumed to be equal to the frequency at which the renewable energy profile is updated. This is only an example.

The frequency of resetting the target net direct hydrogen flow for the hydrogen feed 116, and subsequently the target direct hydrogen flow in the hydrogen supply line 115 and the target stored hydrogen flow in hydrogen supply line 114, may depend on the volume of the hydrogen storage unit 112, the nameplate capacity of the methanol synthesis plant 210, and/or the time required for the pressure of the hydrogen storage unit 112 to reach its high or low constraint limit. In examples, subsystem 150 may be configured to perform calculations to determine the magnitude and frequency of change in the maximum target net direct hydrogen flow for the hydrogen feed 116 to the methanol synthesis plant 210 and subsequently in the maximum target direct hydrogen flow in hydrogen supply line 115 and the maximum target stored hydrogen flow in hydrogen supply line 114, which may be based on thermodynamics and first principles.

In examples, subsystem 150 is configured to predict the mass and pressure of the hydrogen in the hydrogen storage unit 112 over the time horizon for which the renewable power availability profile is given and may use this information to establish the maximum target net hydrogen flow for the hydrogen feed 116 to the methanol synthesis plant 210 and subsequently the target direct hydrogen flow in hydrogen supply line 115 and the target stored hydrogen flow in hydrogen supply line 114.

FIG. 5 illustrates a flowchart 520 of an example of a method that may be carried out by subsystem 150 as described in more detail below.

In examples, subsystem 150 is configured to perform iterative calculations using different assumed values for the target net hydrogen flow for the hydrogen feed 116 to the methanol synthesis plant 210, spanning the range between the turndown capacity of the methanol synthesis plant 210 to its full name plate capacity. At each iterative calculation the target net direct hydrogen flow for the hydrogen feed 116 may be incrementally increased or decreased by a predetermined amount, which may depend on the capacity of the methanol synthesis plant 210.

In examples, subsystem 150 receives one or more of the following inputs 522 to use at each iteration of the calculation of the target net direct hydrogen flow for the hydrogen feed 116 to the methanol synthesis plant 210:
∘ the renewable power availability profile over a time horizon, which may be provided by the operator of the low carbon energy source 10,
∘ the pressure of the hydrogen storage unit 112 at the beginning of the same time horizon,
∘ the efficiency of the primary hydrogen source 111, defined as the rate of hydrogen production generated by the primary hydrogen source 111 as a function of available power,
∘ the capacity of hydrogen storage unit 112,
∘ the allowable range of operating pressures of the hydrogen storage unit 112,
∘ an assumed value for the target net hydrogen flow for the hydrogen feed 116 to the methanol synthesis plant 210.

In examples, as illustrated in FIG. 5, at 524, subsystem 150 then determines the target value for the net hydrogen flow for the hydrogen feed 116.

Step 1 - In examples, based on the efficiency of the primary hydrogen source 111 and an assumed value for the target net hydrogen flow for the hydrogen feed 116 to the methanol synthesis plant 210, subsystem 150 determines the mass of the hydrogen produced at each time interval across the duration of the time horizon for which the renewable power generation profile is available. In this manner, subsystem 150 may establish the profile of the hydrogen flow to the hydrogen storage unit 112 from the primary hydrogen source 111 as a function of time for the entire duration of the time period for which the renewable power profile is available.

Step 2 - In examples, subsystem 150 determines the relationship between the pressure and the density of hydrogen in the hydrogen storage unit 112 based on gas law data at 40 deg C and a range of operating pressures between a low limit and a high limit of the hydrogen storage unit 112.

Step 3 - In examples, subsystem 150 determines the density of the hydrogen in the hydrogen storage unit 112 at the beginning of the time horizon for which the renewable power availability profile is given based on the pressure of the hydrogen storage unit 112 at the beginning of the time horizon for which the renewable power availability profile is given. For each iterative calculation of the target net direct hydrogen flow for the hydrogen feed 116 to the methanol synthesis plant 210 subsystem 150 may determine the density of hydrogen in hydrogen storage unit 112 at each time interval spanning the entire time horizon for which the renewable power availability profile is given. In examples, the density of the hydrogen in hydrogen storage unit 112 may be determined based on the net change in hydrogen mass in the hydrogen storage unit 112 and the volume of the hydrogen storage unit 112. In this manner, at the beginning of the time horizon for which the renewable power availability profile is given subsystem 150 may establish a hydrogen density profile in the hydrogen storage unit 112 for the entire time horizon for which the renewable power availability profile is given.

Step 4 - Based on the hydrogen density profile in the hydrogen storage unit 112 as determined in step 3, in examples, subsystem 150 determines the pressure profile of the hydrogen storage unit 112 at each time interval for the entire duration of the time horizon for which the renewable power availability profile is given. In examples, the pressure profile of the hydrogen storage unit 112 may be determined based on regressing gas law data obtained from first principles as described in step 2 above.

In examples, via steps 3 and 4 above subsystem 150 establishes the hydrogen density and pressure profiles in the hydrogen storage unit 112 at each time interval for the entire duration of the time horizon for which the renewable power availability profile is given for each assumed value of the target net hydrogen flow for the hydrogen feed 116 to the methanol synthesis plant 210 at each iteration.

In examples, subsystem 150 repeats steps 1 through 4 above for each successive assumed value for the target net hydrogen flow for the hydrogen feed 116 to the methanol synthesis plant 210. In this manner, subsystem 150 may establish the hydrogen density and pressure profiles in the hydrogen storage unit 112 for the entire duration of the time horizon for which the renewable power availability profile is given.

In examples, based on the derived density and pressure profiles of hydrogen storage unit 112 for the entire duration of the time horizon for which the renewable power availability profile is given, subsystem 150 determines the value of the targe net hydrogen flow of the hydrogen feed 116 for methanol synthesis plant 210 for a given time interval.

In examples, in making this determination, subsystem 150 may consider a safety margin of the hydrogen storage unit 112. In examples, the safety margins of the hydrogen storage unit 112 may be defined by a user and/or be previously uploaded to subsystem 150. In examples, subsystem 150 may apply a safety margin to the high and low operating pressure limits of the hydrogen storage unit 112.

In examples, to determine the value of the target net hydrogen flow of the hydrogen feed 116 for methanol synthesis plant 210 for a given time interval, subsystem 150 may consider at least one of the two criteria described below, for selecting the best target hydrogen flow to the methanol synthesis plant 210.

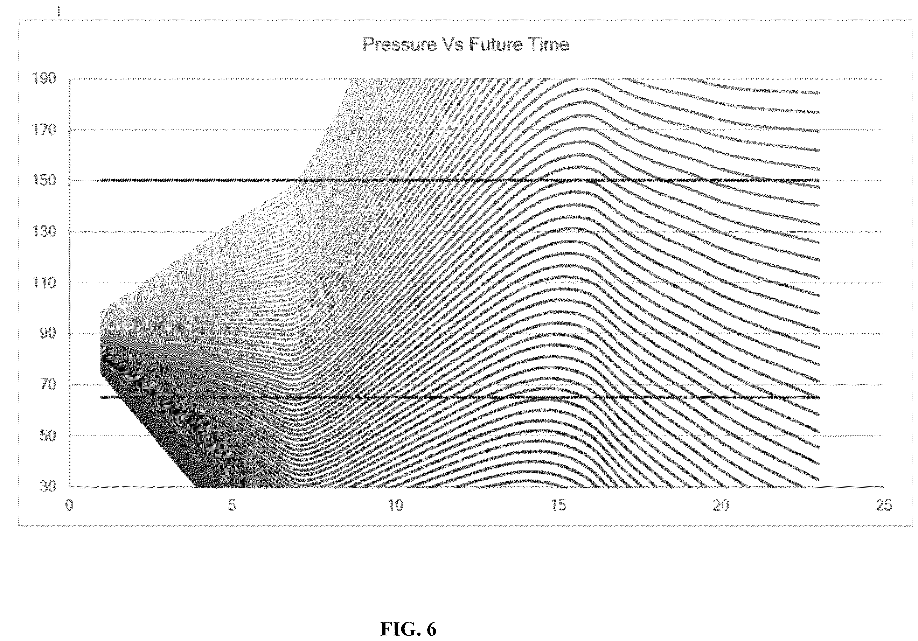

Criterion 1 - In examples, for each iterative target hydrogen flow calculation, subsystem 150 analyzes the pressure profile over the time horizon of the renewable energy profile and may plot the pressure profile across the time horizon, as shown in FIG. 6. In examples, subsystem 150 compares each calculated value of the pressure and compare it to the pressure value of the design high pressure or low-pressure limits of the hydrogen storage unit 112. Subsystem 150 may determine the time required for the pressure of the hydrogen in the hydrogen storage unit 112 to breach the high-pressure or low-pressure limit. In examples, these determined time values may be stored in an array. In examples, subsystem 150 may then select the target net hydrogen flow for the hydrogen feed 116 to the methanol synthesis plant 210 as the maximum value of all elements of the array.

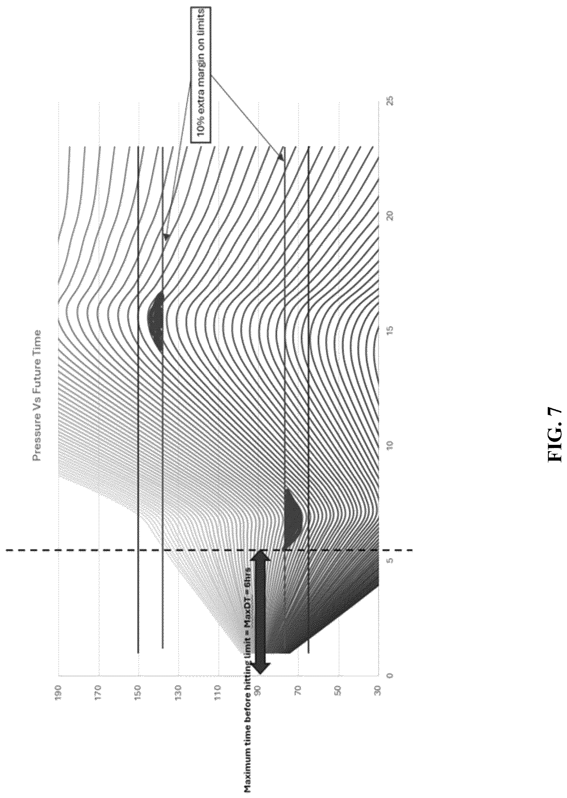

Criterion 2 - In examples, for each iterative calculation using assumed values of target hydrogen flow, subsystem 150 determines the total deviation (area under the curve, shown as shaded area in FIG. 7) of the pressure profile of the hydrogen storage 112 over the time horizon of the renewable power profile from the allowable pressure limits calculated based on a user-defined safety margin (plus or minus) from the design high and low safety pressure limits of the hydrogen storage unit 112. In examples, subsystem 150 stores the calculated values of the total deviation (area under the curve) of each pressure profile from the safety high or low limits in an array. The result of each of these calculations may represent each instance in time when the pressure profile of the hydrogen storage unit 112 intersects high or low safety pressure limits of the hydrogen storage unit 112. In examples, subsystem 150 may determine as the best iterative target net hydrogen flow for the hydrogen feed 116 the one for which the corresponding pressure profile has the lowest deviation (i.e. smallest area under the curve) from the safety limits.

In examples, subsystem 150 selects as the operating target net hydrogen flow to the methanol synthesis plant 210 the greater of the two target hydrogen flows which may have been calculated by criterion 1 and criterion 2.

At 506 (step 2) the calculated average amount of available hydrogen for the forecasted time period may be used as an input to the first principles model. The first principles model may predict the operating conditions of the methanol synthesis plant 210, the primary hydrogen source 111 (e.g. an electrolyzer), the availability of biogenic carbon dioxide feed 215 and non-biogenic carbon dioxide feed 216 and other parts of the plant, for example the hydrogen storage unit 112, etc. corresponding to the calculated average hydrogen target for the entire period of the length of the forecasted energy profile for the low carbon energy source 10.

In examples, at 508 and 510 the ARC 300 determines if the operating conditions predicted at 506 are within the operating limits of each section of facility 100 and within the operating limits of each equipment for the length of the forecasted energy profile for the low carbon energy source 10. Operating limits of each section and/or equipment of facility 100 may be design operating limits set when the facility 100 and/or equipment is designed and/or installed or operating limits defined by the original equipment manufacturer. If the predicted operating conditions are within all operating limits, the ARC 300 may use these operating conditions, which have been validated by the first principles model and may define all the set points 301 for facility 100.

In examples, at 512, the ARC 300 transmits the set points 301 to the DCS 180 to control the operation of facility 100 at the desired target levels.

In examples, at 514, if any of the operating conditions predicted in step 2 are outside the operating limits listed at 508, then at 514 the ARC 300 runs the first principles model iteratively with the purpose to determine the maximum hydrogen rate, which may be consumed by the methanol synthesis plant 210 without causing the operating parameters of any equipment in the plant to violate any of their operating limits. For example, the ARC 300 may try different values for the hydrogen rate, taking into account the forecasted available energy for the low carbon energy source 10. For each of these hydrogen rate values the ARC 300 may determine the operating conditions of facility 100 and select the maximum hydrogen rate that results in desired level of operation of facility 100, which is within the operating limits. At 516, the ARC 300 may use the newly determined maximum hydrogen rate and the predicted operating conditions corresponding to the maximum hydrogen rate to define the set points 301 for facility 100.

When an updated forecasted energy profile for the low carbon energy source may be available and the process including the calculation from 504 to 516 may loop back and be repeated as shown in FIG. 4. In examples, the calculations from 504 to 516 may be repeated at the same frequency as the frequency of the availability of the energy profile for the low or zero carbon energy source 10.

In between the updates of the forecasted energy profile for the low or zero carbon energy source 10, the ARC 300 may monitor continuously the plant conditions and perform the calculations using first principles model described in 502 to 516, in order to determine how it may adjust the set points 301 of facility 100, based on the dynamic relationships between the process variables and the set points 301, such that no process conditions will violate the allowable limits even in situations when the actual weather conditions do not support the forecasted power availability profile. This dynamic relationship may be established based on the first principles model related to the facility 100 or by performing perturbation tests on the operation of facility 100, for example changing the rate of the direct hydrogen feed 115 to the methanol synthesis plant 210 and measuring the impact on the pressure of the hydrogen storage unit 112.

The ARC 300 may be configured to autonomously transmit the set points 301 to the DCS 400. Alternatively, the ARC 300 may be configured to display 'prompts' to a human operator, who will then implement the controls. The ARC 300 may also be configured to operate in a semi-autonomous fashion in which some controls are executed autonomously and others are done by a human operator.

As noted above, a secondary energy source 20 may be used in some situations. The secondary energy source 20 may include an electrical grid. It should be noted that any surplus electricity generated by the low carbon energy source 10 may be fed into the electrical grid through energy connection 14. The secondary energy source 20 may also include a battery bank that is charged by the low carbon energy source 10 and/or the electrical grid through energy connection 14. As noted above, a secondary hydrogen source 30 may be used in some situations. When present, the secondary hydrogen source 30 may provide a supplemental hydrogen feed to the methanol synthesis plant 210. This supplemental hydrogen feed may be in place of or in addition to the supplemental hydrogen feed provided by the stored hydrogen feed 114 from the hydrogen storage unit 112. It should be noted that any surplus hydrogen generated by the primary hydrogen source 111 (e.g. an electrolyzer), or other hydrogen producing equipment, may be fed into the secondary hydrogen source 30 through the secondary hydrogen feed 33 or some other connection line.

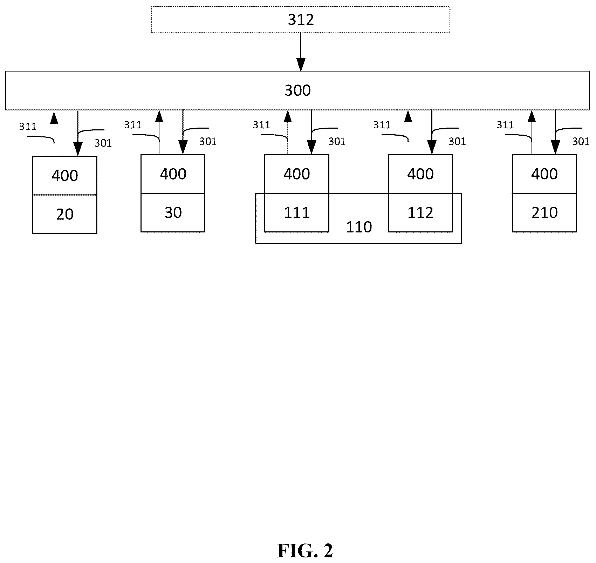

Referring to FIG. 2, there is schematically illustrated a non-limiting embodiment of an ARC 300 in accordance with the present disclosure. The ARC 300 may be used to control various functions of facility 100, including a methanol synthesis plant 210, a secondary energy source 20, a secondary hydrogen source 30, a hydrogen plant 110, and a valve network (not shown). The hydrogen plant 110 includes a primary hydrogen source 111 (e.g. an electrolyzer) and a hydrogen storage unit 112. The valve network (not shown) controls the distribution of hydrogen generated by the primary hydrogen source 111 (e.g. an electrolyzer). The valves (not shown), and associated signal lines, selectively direct the direct hydrogen feed 115 (FIG. 1) to the methanol synthesis plant 210 and storage hydrogen feed 113 (FIG. 1) to the hydrogen storage unit 112.

Each above-listed component includes a DCS 400 that includes logic and hardware designed and configured to control the associated component. Each DCS 400 is also designed and configured to receive and execute one or more set points 301 received from the ARC 300 for the associated component. While all of the DCS 400 are labelled with the same numeral, it should be understood that each DCS 400 may be programmed specifically for the associated component.

The ARC 300 controls one or more operations of facility 100 (FIG. 1) by sending command signals in the form of determined set points 301 to one or more DCS 400. The ARC 300 determines the set point(s) 301 using non-facility-specific information 312 related to the energy profile for the low carbon energy source 10 and/or the energy availability from the secondary energy source 20 (FIG. 1) over a time period. The ARC 300 also receives facility-specific information 311 from one or more of the DCS 400. The facility-specific information 311 may relate to operating parameters discussed previously. Based on the non-facility-specific information 312 and the facility-specific information 311, the ARC 300 generates set point(s) 301 for facility 100 and/or components of facility 100. In examples, the generated set point(s) 301 are transmitted to one or more DCS 400, which control facility 100 and/or components of facility 100.

As shown in FIG. 2, the set point(s) 301 may be transmitted as needed to one or more DCS 400 of the methanol synthesis plant 210, the low carbon energy source 10, the secondary energy source 20, the secondary hydrogen source 30, the primary hydrogen source 111 (e.g. an electrolyzer), the valve network (not shown), and the hydrogen storage unit 112.

In one non-limiting example of operation, as the ARC 300 continuously and/or periodically calculates the energy needs of the entire operation of facility 100, based on facility-specific information 311 and the non-facility-specific information 312, the ARC 300 may determine that the energy profile for the low carbon energy source 10 cannot sustain the operation of the methanol synthesis plant 210 over a period of time. As explained previously, a reduction in electrical energy causes a corresponding reduction in hydrogen production. To mitigate the effect of the loss of available hydrogen, the ARC 300 may utilize the stored hydrogen in the hydrogen storage unit 112. For instance, the ARC 300 may transmit a set point 301 to the DCS 180 to supplement the direct hydrogen feed 115 (FIG. 1) entering the methanol synthesis plant 210 with the stored hydrogen feed 114 (FIG. 1) to compensate for the loss of generated hydrogen. If the amount of stored hydrogen is sufficient to maintain the current flow rate of the direct hydrogen feed 115 (FIG. 1) during the disruption in electricity availability, then the ARC 300 may take no further action.

If the amount of stored hydrogen is insufficient to maintain the desired flow rate of the direct hydrogen feed 115 (FIG. 1) during the disruption, then the ARC 300 may determine a course of action that achieves the operating conditions that optimize methanol production. An exemplary course of action may be to estimate a desirable hydrogen and carbon dioxide flowrate given the predicted amount of available hydrogen. The ARC 300 may then send set point(s) 301 to the DCS 400 (FIG. 2) of facility 100. The set point(s) 301 may periodically or continuously adjust set points at the DCS 400.

In another non-limiting example of operation, the ARC 300 may process the non-facility-specific information 312 and determine no loss of electrical energy availability over a period of time. With no expected need for stored hydrogen, the ARC 300 may direct all hydrogen generated by the primary hydrogen source 111 (e.g. an electrolyzer) to the methanol synthesis plant 210. Thus, energy is not wasted compressing hydrogen for storage.

It should be appreciated that the ARC 300 may also be designed and configured to generate the set points 301 for facility 100 that cause one or more of: (i) send and/or transfer surplus electricity generated by the low carbon energy source 10 to the secondary energy source 20 (FIG. 1) via the energy connection 14 (FIG. 1); (ii) direct a supplemental hydrogen feed from the secondary hydrogen source 30 (FIG. 1) to the methanol synthesis plant 210; and (iii) send and/or transfer surplus hydrogen generated by the hydrogen plant 110 to the secondary hydrogen source 30 (FIG. 1) via the secondary hydrogen feed 115 (FIG. 1) or some other connection line.

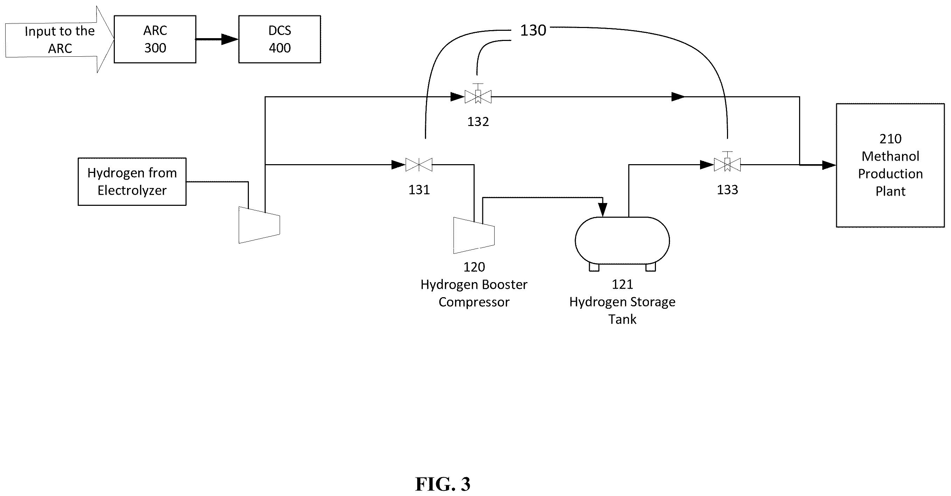

Referring to FIG. 3, there is schematically illustrated a non-limiting embodiment of an ARC 300 in accordance with the present disclosure. The ARC 300 may be configured to control various functions of facility 100, including a low carbon energy source 10, a hydrogen plant 110, and a methanol synthesis plant 210. The hydrogen plant 110 may include a primary hydrogen source 111 (e.g. an electrolyzer) and a hydrogen storage unit 112. The ARC 300 may selectively direct hydrogen flow to the methanol synthesis plant 210 and/or the hydrogen storage unit 112. The hydrogen storage unit 112 may include a compressor 120 and a hydrogen tank 121 and a valve network 130. The valve network 130 may include a flow controller 131 controlling flow into the compressor 120, a bypass flow controller 132 controlling flow along a bypass line and a hydrogen makeup flow controller 133 controlling flow out of the tank along a hydrogen makeup line. The flow controllers 131, 132, 133 may be controlled by the associated DCS 400.

It should be appreciated that electrical energy produced by the low carbon energy source 10 may be in excess of what is required by facility 100. In such instances, the excess energy may be sent to the secondary energy source 20, e.g., utility grid, in which case the secondary energy source 20 functions as an energy sink. Likewise, hydrogen produced by the hydrogen plant 110 in excess of what is required by facility 100. In such instances, the excess hydrogen may be sent to the secondary hydrogen source 30, in which case the secondary hydrogen source 30 functions as a hydrogen sink.

In examples, although not shown, one or more control systems such as, but not limited to, ARC 300 and DCS 400, may each independently include one or more controllers and/or other suitable computing devices may be employed to control one or more of portions of systems described herein. Controllers may include one or more processors and memory communicatively coupled with each other. In the illustrated example, a memory may be used to store logic instructions to operate and/or control and/or monitor the operation of one or more sections and/or equipment or components of facility 100. In examples, the controllers may include or be coupled to input/output devices such as monitors, keyboards, speakers, microphones, computer mouse and the like. In examples, one or more controllers may also include one or more communication components such as transceivers or like structure to enable wired and/or wireless communication. In examples, this may allow for remote operation of one or more systems described herein.

In examples, memory associated with one or more controllers and/or other suitable computing devices may be non-transitory computer-readable media. The memory may store an operating system and one or more software applications, instructions, programs, and/or data to implement the methods described herein and the functions attributed to the various systems. In various implementations, the memory may be implemented using any suitable memory technology, such as static random-access memory (SRAM), synchronous dynamic RAM (SDRAM), nonvolatile/Flash-type memory, or any other type of memory capable of storing information. The controls systems may include any number of logical, programmatic, and physical components.

Logic instructions may include one or more software modules and/or other sufficient information for autonomous operation, safety procedures, and routine maintenance processes. Any operation of the described system may be implemented in hardware, software, or a combination thereof. In the context of software, operations represent computer-executable instructions stored on one or more computer-readable storage media that, when executed by one or more processors, perform the recited operations. Generally, computer-executable instructions include routines, programs, objects, components, data structures, and the like that perform one or more functions or implement particular abstract data types.

It should be noted that the equipment, devices, components, and systems described above are only exemplary of the equipment, devices, components, and systems designed and configured to perform their respective tasks. For example, an electrolyzer is only one example of a device that may be used to generate hydrogen. Other hydrogen sources may utilize renewable liquid reforming, high-temperature water-splitting, photobiological water splitting, photoelectrochemical water splitting, etc. Thus, the present teachings are neither limited to the equipment, devices, components, and systems described above nor the processes used therein.

As used herein, the term "low carbon energy" refers to an energy source that does not use hydrocarbons as the principal source of energy. Examples of low carbon power sources include, but are not limited to, solar power, wind power, tidal power, geothermal power, hydroelectric power, nuclear power, and hydrogen pipeline. It should be noted that the term "low carbon energy" source encompasses power sources that do not emit any carbon, i.e., "a zero carbon energy source."

The words "comprising" and "comprises" as used throughout the claims, are to be interpreted to mean "including but not limited to" and "includes but not limited to", respectively.

To the extent used herein, the word "substantially" shall mean "being largely but not wholly that which is specified."

As used herein, the singular forms "a," "an," and "the" are intended to include the plural forms as well, unless the context clearly indicates otherwise.

To the extent used herein, the term "about" in reference to a given parameter is inclusive of the stated value and has the meaning dictated by the context (e.g., it includes the degree of error associated with measurement of the given parameter).

To the extent used herein, the term "and/or" includes any and all combinations of one or more of the associated listed items.

The foregoing description is directed to particular embodiments of the present disclosure for the purpose of illustration and explanation. It will be apparent, however, to one skilled in the art that many modifications and changes to the embodiment set forth above are possible without departing from the scope of the disclosure. It is intended that the following claims be interpreted to embrace all such modifications and changes.

The present disclosure is further illustrated by the following items:
Item 1. A method for producing methanol using a low carbon energy source,
   the methanol being produced by a facility having a methanol synthesis plant, the method comprising:
   supplying energy to the facility, wherein at least a portion of the supplied energy is from a low carbon energy source;
   receiving a forecasted energy profile for the low carbon energy source over a time period;
   predicting, using an advanced regulatory controller (ARC), operating conditions of the facility based on the forecasted energy profile for the low carbon energy source;
   generating, by the ARC, one or more set points to control the facility;
   generating a hydrogen feed to the methanol synthesis plant using at least one of:
      (i) a primary hydrogen feed generated by a hydrogen plant energized by the low carbon energy source, and
      (ii) a supplemental hydrogen feed;
   controlling the generating of the hydrogen feed using the ARC; and
   producing methanol by feeding the generated hydrogen feed to the methanol synthesis plant in accordance with one or more set points generated by the ARC.
Item 2. The method of item 1, wherein generating the hydrogen feed comprises using the supplemental hydrogen feed provided from: (i) a hydrogen storage unit energized by the low carbon energy source, (ii) a secondary hydrogen source, or (iii) a combination of (i) and (ii).
Item 3. The method of item 1, wherein the one or more set points for the facility are generated using facility-specific information and non-facility-specific information, the non-facility-specific information comprising the forecasted energy profile of the low carbon energy source.
Item 4. The method of item 3, wherein the non-facility-specific information further comprises availability of energy from a secondary energy source, and further comprising transferring energy between the facility and the secondary energy source.
Item 5. The method of item 4, wherein the transferring comprises one of: (i) exporting energy from the low carbon energy source to the secondary energy source, and (ii) importing energy from the secondary energy source to the facility.
Item 6. The method of item 1, wherein the forecasted energy profile for the low carbon energy source is received continuously or periodically.
Item 7. The method of item 6, wherein one or more set points are generated by the ARC each time an update to the energy profile for the low carbon energy source is received.
Item 8. The method of item 1, further comprising determining if the predicted operating conditions of the facility are outside operating limits of one or more sections or equipment of the facility.
Item 9. The method of item 8, wherein generating the one or more set points for the facility further comprises:
   determining that the predicted operating conditions of the facility are outside the operating limits of one or more sections or equipment of the facility;
   determining, in response to the determination that the predicted operating conditions are outside the operating limits, a maximum hydrogen rate that may be consumed by the methanol synthesis plant without violating any of the operating limits of any equipment and/or process in the facility; and
   generating the one or more set points for the facility based on the determined maximum hydrogen rate and on the operating limits of one or more sections or equipment of the facility.
Item 10. The method of item 1, further comprising:
   converting the forecasted energy profile for the low carbon energy source into a net target hydrogen flow amount;
   wherein predicting operating conditions is based at least in part on the net target hydrogen flow amount; and
   wherein the one or more set points are generated based at least in part on the operation conditions predicted based on the net target hydrogen flow amount.
Item 11. A control system for producing methanol using a low carbon energy source, the methanol being produced by a facility having a methanol synthesis plant, the control system comprising:
   an advanced regulatory controller (ARC) configured to:
   receive a forecasted energy profile for the low carbon energy source over a time period;
   predict operating conditions of the facility based on the forecasted energy profile for the low carbon energy source; and
   generate one or more set points for the facility and to control the generation of a hydrogen feed to the methanol synthesis plant for production of the methanol.
Item 12. The control system of item 11, wherein the one or more set points are generated based on the received forecasted energy profile for the low carbon energy source.
Item 13. The control system of item 11, wherein the ARC is further configured to:
   receive a level of energy availability from a secondary energy source.
Item 14. The control system of item 13, wherein the ARC is further configured to:
   cause a transfer of energy between the facility and the secondary energy source.
Item 15. The control system of item 11, wherein the ARC is further configured to:
   determine if the predicted operating conditions of the facility are outside operating limits of one or more sections or equipment of the facility;
   determine, in response to the determination that the predicted operating conditions of the facility are outside the operating limits, a maximum hydrogen rate that may be consumed by the methanol synthesis plant without violating any of the operating limits of any equipment and/or process in the facility; and
   use the maximum hydrogen rate and on the operating limits of one or more sections or equipment of the facility to generate one or more set points.
Item 16. The control system of item 11, further comprising one or more distributed control systems configured to receive the one or more set points generated by the ARC.
Item 17. The control system of item 11, wherein the ARC further comprises a subsystem configured to:
   convert the forecasted energy profile for the low carbon energy source into a net target hydrogen flow amount;
   wherein the operating conditions are predicted based on the net target hydrogen flow amount; and
   wherein the one or more set points are generated based on the operation conditions predicted based on the net target hydrogen flow amount.
Item 18. A non-transitory computer readable medium having stored thereon computer-readable instructions that, when executed by a processor, cause the processor to:
   receive facility-specific information and non-facility-specific information, wherein the non-facility-specific information comprises a forecasted energy profile for a low carbon energy source over a time period;
   predict operating conditions of the facility based on the forecasted energy profile for the low carbon energy source;
   determine if the predicted operating conditions of the facility are outside operating limits of one or more sections or equipment of the facility;
   generate, based on the determination, one or more set points for the facility; and
   control production of methanol based on one or more set points.
Item 19. The non-transitory computer readable medium of item 18, further configured to continuously monitor one or more components of a facility comprising a methanol synthesis plant and the low carbon energy source.
Item 20. The non-transitory computer readable medium of item 19, further configured to receive a level of energy availability from a secondary energy source.
Item 21. The non-transitory computer readable medium of item 20, further configured to cause a transfer of energy from the low carbon energy source to a secondary source of energy.
Item 22. The non-transitory computer readable medium of item 18 further configured to:
   convert the forecasted energy profile for the low carbon energy source into a net target hydrogen flow amount;
   wherein the operating conditions are predicted based at least in part on the net target hydrogen flow amount; and
   wherein the one or more set points are generated based at least in part on the operation conditions predicted based on the net target hydrogen amount.
Item 23. A method for controlling a facility for producing methanol using a low carbon energy source and a methanol synthesis plant, comprising:
   receiving facility-specific information and non-facility-specific information, wherein the non-facility-specific information comprises a forecasted energy profile for a low carbon energy source over a time period;
   predicting operating conditions of the facility based on the forecasted energy profile for the low carbon energy source;
   determining if the predicted operating conditions of the facility are outside operating limits of one or more sections or equipment of the facility;
   generating, based on the determination, one or more set points for the facility; and
   controlling production of methanol based on one or more set points.
Item 24. The method of item 23, further comprising:
   converting the forecasted energy profile for the low carbon energy source into a net target hydrogen flow amount;
   wherein the operating conditions are predicted based at least in part on the net target hydrogen flow amount; and
   wherein the one or more set points are generated based at least in part on the operation conditions predicted based on the net target hydrogen flow amount.

## Claims

1. A method for producing methanol using a low carbon energy source, the methanol being produced by a facility having a methanol synthesis plant, the method comprising:
supplying energy to the facility, wherein at least a portion of the supplied energy is from a low carbon energy source;
receiving a forecasted energy profile for the low carbon energy source over a time period;
predicting, using an advanced regulatory controller (ARC), operating conditions of the facility based on the forecasted energy profile for the low carbon energy source;
generating, by the ARC, one or more set points to control the facility;
generating a hydrogen feed to the methanol synthesis plant using at least one of:
(i) a primary hydrogen feed generated by a hydrogen plant energized by the low carbon energy source, and
(ii) a supplemental hydrogen feed;
controlling the generating of the hydrogen feed using the ARC; and
producing methanol by feeding the generated hydrogen feed to the methanol synthesis plant in accordance with one or more set points generated by the ARC.

2. The method of claim 1, wherein generating the hydrogen feed comprises using the supplemental hydrogen feed provided from: (i) a hydrogen storage unit energized by the low carbon energy source, (ii) a secondary hydrogen source, or (iii) a combination of (i) and (ii).

3. The method of claim 1, wherein the one or more set points for the facility are generated using facility-specific information and non-facility-specific information, the non-facility-specific information comprising the forecasted energy profile of the low carbon energy source.

4. The method of claim 3, wherein the non-facility-specific information further comprises availability of energy from a secondary energy source, and further comprising transferring energy between the facility and the secondary energy source, optionally
wherein the transferring comprises one of: (i) exporting energy from the low carbon energy source to the secondary energy source, and (ii) importing energy from the secondary energy source to the facility.

5. The method of claim 1, wherein the forecasted energy profile for the low carbon energy source is received continuously or periodically, optionally
wherein one or more set points are generated by the ARC each time an update to the energy profile for the low carbon energy source is received.

6. The method of claim 1, further comprising determining if the predicted operating conditions of the facility are outside operating limits of one or more sections or equipment of the facility, optionally
wherein generating the one or more set points for the facility further comprises:
determining that the predicted operating conditions of the facility are outside the operating limits of one or more sections or equipment of the facility;
determining, in response to the determination that the predicted operating conditions are outside the operating limits, a maximum hydrogen rate that may be consumed by the methanol synthesis plant without violating any of the operating limits of any equipment and/or process in the facility; and
generating the one or more set points for the facility based on the determined maximum hydrogen rate and on the operating limits of one or more sections or equipment of the facility.

7. The method of claim 1, further comprising:
converting the forecasted energy profile for the low carbon energy source into a net target hydrogen flow amount;
wherein predicting operating conditions is based at least in part on the net target hydrogen flow amount; and
wherein the one or more set points are generated based at least in part on the operation conditions predicted based on the net target hydrogen flow amount.

8. A control system for producing methanol using a low carbon energy source, the methanol being produced by a facility having a methanol synthesis plant, the control system comprising:
an advanced regulatory controller (ARC) configured to:
receive a forecasted energy profile for the low carbon energy source over a time period;
predict operating conditions of the facility based on the forecasted energy profile for the low carbon energy source; and
generate one or more set points for the facility and to control the generation of a hydrogen feed to the methanol synthesis plant for production of the methanol.

9. The control system of claim 8, wherein the one or more set points are generated based on the received forecasted energy profile for the low carbon energy source,
or wherein the ARC is further configured to:
receive a level of energy availability from a secondary energy source, optionally wherein the ARC is further configured to: cause a transfer of energy between the facility and the secondary energy source;
or
wherein the ARC is further configured to:
determine if the predicted operating conditions of the facility are outside operating limits of one or more sections or equipment of the facility;
determine, in response to the determination that the predicted operating conditions of the facility are outside the operating limits, a maximum hydrogen rate that may be consumed by the methanol synthesis plant without violating any of the operating limits of any equipment and/or process in the facility; and
use the maximum hydrogen rate and on the operating limits of one or more sections or equipment of the facility to generate one or more set points;
or
further comprising one or more distributed control systems configured to receive the one or more set points generated by the ARC;
or
wherein the ARC further comprises a subsystem configured to:
convert the forecasted energy profile for the low carbon energy source into a net target hydrogen flow amount;
wherein the operating conditions are predicted based on the net target hydrogen flow amount; and
wherein the one or more set points are generated based on the operation conditions predicted based on the net target hydrogen flow amount.

10. A non-transitory computer readable medium having stored thereon computer-readable instructions that, when executed by a processor, cause the processor to:
receive facility-specific information and non-facility-specific information, wherein the non-facility-specific information comprises a forecasted energy profile for a low carbon energy source over a time period;
predict operating conditions of the facility based on the forecasted energy profile for the low carbon energy source;
determine if the predicted operating conditions of the facility are outside operating limits of one or more sections or equipment of the facility;
generate, based on the determination, one or more set points for the facility; and
control production of methanol based on one or more set points.

11. The non-transitory computer readable medium of claim 10, further configured to continuously monitor one or more components of a facility comprising a methanol synthesis plant and the low carbon energy source.

12. The non-transitory computer readable medium of claim 11, further configured to receive a level of energy availability from a secondary energy source, optionally
further configured to cause a transfer of energy from the low carbon energy source to a secondary source of energy.

13. The non-transitory computer readable medium of claim 10 further configured to:
convert the forecasted energy profile for the low carbon energy source into a net target hydrogen flow amount;
wherein the operating conditions are predicted based at least in part on the net target hydrogen flow amount; and
wherein the one or more set points are generated based at least in part on the operation conditions predicted based on the net target hydrogen amount.

14. A method for controlling a facility for producing methanol using a low carbon energy source and a methanol synthesis plant, comprising:
receiving facility-specific information and non-facility-specific information, wherein the non-facility-specific information comprises a forecasted energy profile for a low carbon energy source over a time period;
predicting operating conditions of the facility based on the forecasted energy profile for the low carbon energy source;
determining if the predicted operating conditions of the facility are outside operating limits of one or more sections or equipment of the facility;
generating, based on the determination, one or more set points for the facility; and
controlling production of methanol based on one or more set points.

15. The method of claim 14, further comprising:
converting the forecasted energy profile for the low carbon energy source into a net target hydrogen flow amount;
wherein the operating conditions are predicted based at least in part on the net target hydrogen flow amount; and
wherein the one or more set points are generated based at least in part on the operation conditions predicted based on the net target hydrogen flow amount.
